# EUROPÄISCHE PATENTSCHRIFT

(11) **EP 0 775 106 B1**
(45) Veröffentlichungstag und Bekanntmachung des Hinweises auf die Patenterteilung: **20.01.1999**
(21) Anmeldenummer: 95927718.7
(22) Anmeldetag: 25.07.1995
(51) Int. Cl.: C07C 249/12, C07C 251/54

(54) **VERFAHREN ZUR HERSTELLUNG VON O-(2-HYDROXYALKYL)-OXIMEN**
METHOD OF PRODUCING O-(2-HYDROXYALKYL)-OXIMES
PROCEDE DE PRODUCTION DE 0-(2-HYDROXYALKYL)-OXIMES

(30) Priorität: 02.08.1994 DE 4427290
(43) Veröffentlichungstag der Anmeldung: 28.05.1997
(73) Patentinhaber: BASF AKTIENGESELLSCHAFT, 67056 Ludwigshafen (DE)
(72) Erfinder: HARREUS, Albrecht, D-67063 Ludwigshafen (DE); GÖTZ, Norbert, D-67547 Worms (DE); RANG, Harald, D-67122 Altrip (DE)
(86) Internationale Anmeldenummer: EP9502932
(87) Internationale Veröffentlichungsnummer: WO9604237

(56) Entgegenhaltungen:
- EP-A- 0 023 560
- DE-A- 4 415 887
- FR-A- 2 325 626
- JOURNAL OF MEDICINAL CHEMISTRY, Bd.27, Nr.10, Oktober 1984, WASHINGTON US Seiten 1291 - 4 M. BOUZOUBAA ET. AL. 'Synthesis and beta-Adrenergic Blocking Activity of New Aliphatic and alicyclic Oxime Ethers.'

## Beschreibung

Die vorliegende Erfindung betrifft ein Verfahren zur Herstellung von O-(2-Hydroxyalkyl)-oximen der allgemeinen Formel I 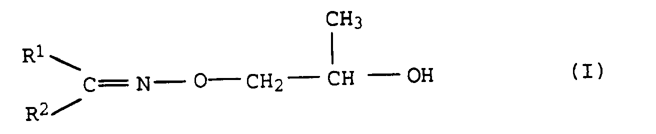 in der R¹ und R² jeweils für Alkylgruppen mit 1 bis 10 Kohlenstoffatomen stehen oder zusammen mit dem Kohlenstoffatom, das sie tragt, zu einem 5- bis 7-gliedrigen Cycloalkylrest verbunden sind.

O-(2-Hydroxyalkyl)-oximen kommt große Bedeutung als Zwischenprodukte für Pflanzenschutzwirkstoffe zu (vgl. z.B. die ältere deutsche Anmeldung DE-A 44 15 887).

Bei der Herstellung von Verbindungen des Typs I durch O-Alkylierung von Hydroxylaminen entsteht immer zu einem gewissen Grad auch die zu I isomere Verbindung, welche die CH₃-Gruppe am C-Atom, das dem Oxim-Sauerstoff benachbart ist, trägt.

Die bisher beschriebenen Hydrierungen von Oxiranen führen entweder zum primären Alkohol oder zu einem Gemisch aus sekundärem und primärem Alkohol (vgl. z.B. US 4,064,186 und Houben-Weyl, Methoden der Organischen Chemie, Georg Thieme Verlag Stuttgart, Bd. 6/3, 4. Auflage 1965, Seiten 442-446).

Aufgabe der Erfindung war es daher, ein Verfahren bereitzustellen, das mit hoher Regioselektivität und weitgehend ohne Bildung von Nebenprodukten zu den O-(2-Hydroxyalkyl)-oximen I führt.

Demgemäß wurde ein Verfahren zur Herstellung der O-(2-Hydroxyalkyl)-oxime der Formel I gefunden, welches dadurch gekennzeichnet ist, daß man ein O-(2,3-Epoxyalkyl)-oxim der allgemeinen Formel II 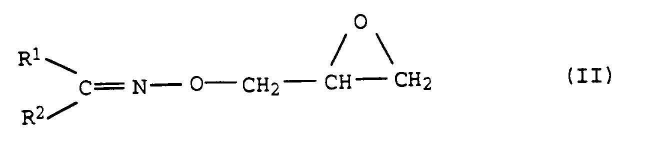 mit Wasserstoff katalytisch hydriert.

Im folgenden beziehen sich die Literaturzitate "Houben-Weyl" auf: Houben-Weyl, Methoden der Organischen Chemie, 4. Auflage, Thieme Verlag, Stuttgart.

Unter den nach dem erfindungsgemäßen Verfahren herstellbaren Verbindungen I sind jene bevorzugt, in denen R¹ und R² jeweils für eine C₁-C₄-Alkylgruppe und vor allem eine C₁-C₃-Alkylgruppe stehen, oder zusammen mit dem Kohlenstoffatom, das sie trägt, einen Cyclopentyl- oder Cyclohexylring bilden. Besonders bevorzugt stehen R¹ und R² jeweils für Methyl oder Ethyl, insbesondere beide für Methyl.

Die O-(2,3-Epoxyalkyl)-oxime II sind allgemein bekannt oder nach bekannten Methoden, vor allem durch basenkatalysierte Umsetzung der entsprechenden Epihalogenhydrine mit den freien Oximen in einem dipolar aprotischen Lösungsmittel, erhältlich (vgl. z.B. Zh. Org. Khim. 5, Seiten 1353 bis 1355 (1969)).

Als Katalysatoren für die Hydrierung eignen sich hierfür allgemein übliche Katalysatoren, wie sie z.B. in "Houben-Weyl", Band 4/1c, beschrieben sind.

Bevorzugte Hydrierkatalysatoren sind solche, die ein Metall aus den Gruppen 8 bis 10 gemäß der IUPAC-Einteilung des Periodensystems, vorzugsweise aus der Gruppe der Elemente Cobalt, Ruthenium, Rhodium, vor allem aus der Gruppe der Elemente Platin und Nickel und insbesondere Palladium enthalten.

Die Katalysatoren können als solche oder vorzugsweise auf einem Träger verwendet werden. Es kommen übliche Trägermaterialien wie Siliciumdioxid, Aluminiumoxid, Titandioxid, Silikate und Zeolithe und vor allem Aktivkohle in Betracht.

Zur Herstellung der Trägerkatalysatoren können Bindemittel oder Formhilfsmittel mitverwendet werden. Die Katalysatoren können in Form von Splitt, Strängen, Tabletten oder Kugeln eingesetzt werden.

Pro Mol der zu hydrierenden Verbindung II werden im allgemeinen 0,1 bis 2, vorzugsweise 0,1 bis 1 Gew.-% Katalysator eingesetzt, wobei sich diese Mengenangabe auf die aktive Masse des Katalysators ohne Trägermaterialien bezieht.

Die Hydrierung kann kontinuierlich oder vorzugsweise diskontinuierlich durchgeführt werden.

Bei der diskontinuierlichen Durchführung in der Flüssigphase kann die Hydrierung in Gegenwart eines Lösungsmittels erfolgen. Geeignet sind polare Lösungsmittel wie Ether und Alkohole sowie Mischungen hiervon. Bevorzugt sind Ether oder Alkohole mit bis zu sechs Kohlenstoffatomen wie 1,2-Dimethoxyethan, Ethanol, n-Propanol, iso-Propanol und n-Butanol.

Der Druck ist in weiten Grenzen wählbar, die von 1 bis 400 bar reichen, jedoch arbeitet man vorzugsweise in einem Druckbereich von 1 bis 100, vor allem von 10 bis 70 und insbesondere von 30 bis 50 bar.

Die Temperatur bei der Hydrierung beträgt vorzugsweise 0 bis 100, vor allem 10 bis 50 und insbesondere 25 bis 30°C.

Die Isolierung der Reaktionsprodukte I geschieht nach an sich bekannten Methoden, vorzugsweise destillativ. Die Ausbeute an den Verbindungen I liegen normalerweise bei 70 bis 90 %, bei einer Selektivität von in der Regel über 98 %.

Die nach dem erfindungsgemäßen Verfahren erhältlichen O-(2-Hydroxyalkyl)-oxime I eignen sich als Vorprodukte für Herbizide, insbesondere vom Cyclohexenontyp (vgl. z.B. DE-A 44 15 887).

### Beispiel Herstellung von O-(2-Hydroxypropyl)-propan-2-onoxim

130 g (0,94 mol) O-(2,3-Epoxypropyl)-propan-2-onoxim (vgl. EP-A 23 560; Zh. Org. Khim. 5, Seiten 1353 bis 1355 (1969)) und 600 ml Ethanol wurden mit 4 g Palladium/Kohle (10 Gew.-% Palladium) versetzt und bei 25°C und einem Wasserstoffdruck von 50 bar hydriert. Nach 17 Stunden war die Umsetzung vollständig. Der Katalysator wurde abfiltriert. Danach wurde das Lösungsmittel entfernt und der Rückstand über eine Füllkörperkolonne von 50 cm Länge und 5 cm Durchmesser destilliert, welche mit Maschendrahtringen von 3 mm Durchmesser gefüllt war (Kp.: 74°C/20 mbar).

Die Ausbeute an O-(2-Hydroxypropyl)-propan-2-onoxim betrug 84 %, bei einer Reinheit von 99,8 %. Isomeres O-(2-Hydroxy-1-methyl-ethyl)-propan-2-onoxim war dabei nicht entstanden.

## Patentansprüche

1. Verfahren zur Herstellung von O-(2-Hydroxyalkyl)-oximen der allgemeinen Formel I 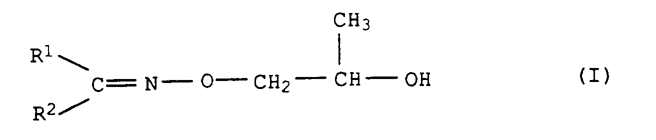 in der R¹ und R² jeweils für Alkylgruppen mit 1 bis 10 Kohlenstoffatomen stehen oder zusammen mit dem Kohlenstoffatom, das sie trägt, zu einem 5- bis 7-gliedrigen Cycloalkylrest verbunden sind, dadurch gekennzeichnet, daß man ein O-(2,3-Epoxyalkyl)-oxim der allgemeinen Formel II 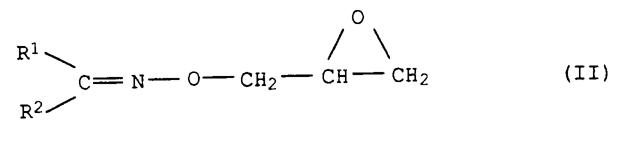 mit Wasserstoff katalytisch hydriert.

2. Verfahren nach Anspruch 1, dadurch gekennzeichnet, daß man von einem O-(2,3-Epoxyalkyl)-oxim ausgeht, in dem R¹ und R² Methyl bedeuten.

3. Verfahren nach Anspruch 1 oder 2, dadurch gekennzeichnet, daß man als Katalysator ein Element aus den Gruppen 8 bis 10 des Periodensystems verwendet.

4. Verfahren nach den Ansprüchen 1 bis 3, dadurch gekennzeichnet, daß man als Katalysator Palladium, gewünschtenfalls auf einem Träger, verwendet.

## Claims

1. A process for preparing O-(2-hydroxyalkyl)oximes of the general formula I 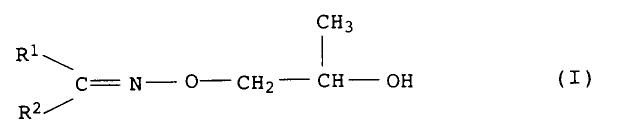 where R¹ and R² in each case are alkyl groups having 1 to 10 carbon atoms or, together with the carbon atom which carries them, are bonded to give a 5- to 7-membered cycloalkyl radical, which comprises catalytically hydrogenating an O-(2,3-epoxyalkyl)oxime of the general formula II 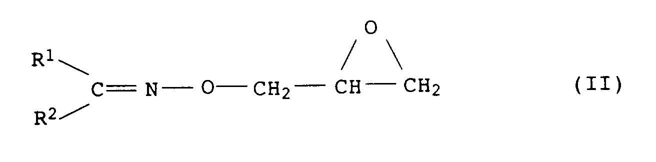 using hydrogen.

2. A process as claimed in claim 1, wherein the starting material used is an O-(2,3-epoxyalkyl)oxime where R¹ and R² are methyl.

3. A process as claimed in claim 1 or 2, wherein the catalyst used is an element of groups 8 to 10 of the Periodic Table.

4. A process as claimed in claims 1 to 3, wherein the catalyst used is palladium, if desired on a support.

## Revendications

1. Procédé pour la préparation d'O-(2-hydroxyalkyl)oximes de formule générale I 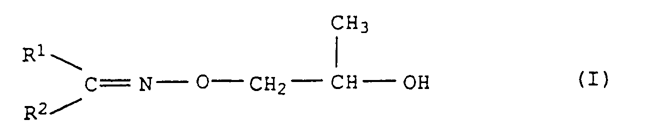 dans laquelle R¹ et R² représentent chacun un groupe alkyle en C1-C10 ou forment ensemble et avec l'atome de carbone auquel ils sont reliés un groupe cycloalkyle de cinq à sept chaînons, ce procédé se caractérisant par le fait que l'on soumet une O-(2,3-époxyalkyl)oxime de formule générale II 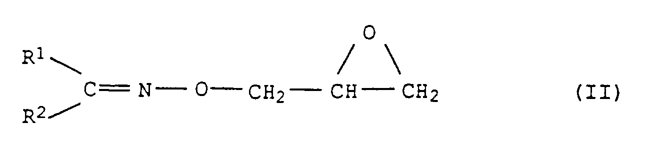 à hydrogénation catalytique.

2. Procédé selon la revendication 1, caractérisé par le fait que l'on part d'une O-(2,3-époxyalkyl)oxime pour laquelle R¹ et R² représentent des groupes méthyle.

3. Procédé selon la revendication 1 ou 2, caractérisé par le fait que l'on utilise, en tant que catalyseur, un élément des groupes 8 à 10 de la Classification Périodique.

4. Procédé selon les revendications 1 à 3, caractérisé par le fait que le catalyseur utilisé est le palladium, éventuellement sur support.
